# EUROPEAN PATENT APPLICATION

(11) **EP 4 464 308 A1**
(43) Date of publication of application: **20.11.2024**
(21) Application number: 24174452.3
(22) Date of filing: 07.05.2024
(51) Int. Cl.: A61K 8/362, A61K 8/365, A61K 8/44, A61K 8/73, A61Q 19/08

(54) **COMPOSITIONS CONTAINING HYALURONIC ACID, AMINO ACIDS AND KREBS CYCLE INTERMEDIATES USEFUL TO INCREASE THE GENE EXPRESSION OF THE EXTRACELLULAR MATRIX**

(30) Priority: 10.05.2023 IT 202300009351
(71) Applicant: Professional Dietetics S.p.A., 20129 Milano (MI) (IT)
(72) Inventor: GIORGETTI, Paolo, 20129 MILANO (IT)
(74) Representative: Bianchetti & Minoja with Trevisan & Cuonzo IPS SRL

(57) **Abstract**

The invention relates to compositions for use in dermatology and cosmetics, comprising a mixture of amino acids, hyaluronic acid or salts thereof and Krebs cycle acids, in particular malic and succinic acid or salts thereof. The compositions according to the invention are useful to counteract skin aging caused by a decline in extracellular matrix deposition.

## Description

The invention relates to compositions for use in dermatology and cosmetics, comprising a mixture of hyaluronic acid, amino acids or salts thereof and Krebs cycle acids, in particular malic and succinic acid or salts thereof. The compositions according to the invention are useful to counteract skin aging caused by a decline in extracellular matrix deposition.

### Prior art

In the human body, the skin is one of the organs most affected by the aging process. Nutritional approaches designed to counteract the age-associated decline in extracellular matrix (ECM) deposition may represent a useful tool for reducing the degenerative processes underlying skin aging.

Skin aging is characterised by sequential, cumulative alterations in the structure and function of the skin, mainly attributable to a reduction in production of its extracellular matrix (ECM) component (Shin et al., 2019; Sparavigna, 2020). The decline in ECM function causes loss of skin elasticity and resilience and the appearance of wrinkles, which is a typical characteristic of skin aging (Black et al., 2008; Sparaviga, 2020, Birch, 2018). Fibroblasts produce ECM mainly consisting of proteins and glycosaminoglycans, the main constituent whereof is hyaluronic acid (HA), the key molecule involved in skin hydration, due to its ability to bind and retain water molecules; a reduction in the content and activity of dermal HA during aging, deriving from both reduced synthesis and increased degradation by tissue hyaluronidase (HAse), therefore leads to dryness and loss of hydration of the skin (Papakonstantinou et al., 2012; Scarano et al., 2021).

The protein component of ECM comprises fibrous proteins such as collagen, elastin, fibronectin and laminin, characterised by a well-defined amino-acid (AA) composition. Elastin (ELN) is the main protein responsible for skin elasticity, constituting about 2% of the total dermal proteins (Theocharis et al., 2016). ELN is synthesised via its soluble isoform tropoelastin, formed by alternating hydrophobic and hydrophilic domains. The hydrophobic domains are enriched with non-polar AAs such as glycine, valine, proline and alanine, arranged in repeated sequences of three to nine AAs, while the hydrophilic domains are enriched with alanine and lysine. Fibronectin (Fbn) plays a crucial part in cell bonding, movement and adhesion; Fbn therefore exhibits various bonding patterns in its protein structure, characterised by defined AA sequences such as Arg-Gly-Asp, Arg-Gly-Asp-Ser, Leu-Asp-Val and Arg-Glu-Asp-Val, which mediate the adhesion function of the Fbn cells (Rosso et al., 2004). Collagen (Col) is the most abundant protein in mammals, and represents the main component of extracellular matrix (ECM), up to 75% in the dermis. Col has a triple-helix structure deriving from the molecular bond conferred by a rigid AA sequence, with repeating glycine-X-Y patterns, wherein glycine stabilises the triple helix, and X and Y are often proline or hydroxyproline; therefore, similarly to ELN and Fbn, Col exhibits a precise AA composition with a high percentage of glycine, hydroxyproline, proline and alanine. The collagen family comprises 28 members, numbered I to XXVIII; however, there are different isoforms in the same type of collagen, and the use of alternative promoters and other proteolytic cleavages gives rise to further levels of diversity in the Col protein family (Vuorio and De Crombrugghe, 1990; Langton et al., 2010; Chu, 2011; Dalton and Lemmon, 2021).

In addition to the decline in the structural components of ECM, excessive production of reactive oxygen species (ROS) is another critical factor in skin aging. According to Harman's theory of aging, said process is mainly attributable to a decline in mitochondrial function, which leads to an increase in ROS generation as by-products of aerobic respiration; oxidative stress is therefore the factor mainly responsible for premature skin aging due to UV, which damages mitochondrial DNA (mtDNA), causes an oxidative phosphorylation (OXPHOS) dysfunction, and increases ROS production (Ziada et al., 2020).

Currently, methods designed to delay skin aging are based on preventive approaches such as avoiding excessive exposure to the sun and/or the use of anti-UV sun creams (Mohiuddin, 2019). At the same time, other treatment strategies involve supplementation with natural compounds, such as polyphenols, plant extracts or HA, to attenuate age-associated skin dryness. However, it has been demonstrated that supplementation of fibroblasts with a mixture of six amino acids (glycine, L-proline, L-leucine, L-lysine, L-valine and L-alanine) increases expression of the components of ECM. Moreover, said 6 amino acids induce the expression of antioxidant genes (Tedesco et al., 2022). Both *de novo* protein synthesis and remodelling and renewal of the ECM components, which underlie its ability to sustain mechanotransductive and tensive properties, have a high energy cost, which requires increased production of mitochondrial ATP (Romani al., 2021). Stimulation of mitochondrial activities may therefore help to promote ECM deposition in the fibroblasts and, by improving the aerobic respiration of the skin, counteract oxidative stress-induced skin aging.

### Description of the invention

It has now been discovered that a mixture of six amino acids consisting of glycine, L-proline, L-leucine, L-lysine, L-valine and L-alanine, combined with hyaluronic acid, malic acid, succinic acid or pharmaceutically acceptable salts thereof, exercises synergistic effects on the stimulation of ECM gene expression. The object of the invention is therefore compositions comprising said amino acids combined with hyaluronic acid, malic acid and succinic acid, which are useful to counteract skin aging. For that purpose, the compositions can be conveniently administered by injection, in the form of ready-for-use sterile solutions, or as freeze-dried powders to be reconstituted with sterile water for injection. The compositions can also be in gel form for use as intradermal fillers. Alternatively, the formulations according to the invention can also be used by topical or transdermal administration, for example in the form of ointments, gel, creams and the like.

The weight ratios of the amino acids substantially correspond to the ratios present in collagen and elastin. The reciprocal ratios, using glycine as reference, are preferably as follows.
- Glycine 1;
- L-proline: 0.7-0.8%, preferably 0.75.
- L-alanine: 0.47-0.76, preferably 0.48-0.51, more preferably 0.75-0.76;
- L-valine: 0.35-0.56; preferably 0.35-0.37 or 0.54-0.56;
- L-leucine: 0.13-0.27; preferably 0.13-0.15.
- L-lysine hydrochloride:0.10-0.12; preferably 0.10-0.11.

The mixture of amino acids in the ratios specified above is present in the compositions according to the invention at concentrations ranging from 5 to 30%, preferably from 5 to 15%, by weight.

The weight ratios of malic acid and succinic acid are preferably about 1:1. The malic and succinic acids or salts thereof are preferably present at concentrations of 1 to 5% by weight.

The hyaluronic acid or salts thereof, in particular sodium hyaluronate, preferably have an average molecular weight Mn ranging between 50,000 and 2,500,000 Da. The percentages of hyaluronic acid or sodium hyaluronate range between 1 and 5% by weight of the total composition.

For the intended dermatological, cosmetic or therapeutic uses, the compositions according to the invention will be administered subcutaneously or intradermally in amounts ranging from 0.1 to 10 ml, depending on the site of application and the condition to be treated or prevented. In the case of topical administration, the formulations can be applied in amounts ranging from 0.1 to 10 g.

The invention is illustrated in detail in the following examples.

### Example 1

| **INGREDIENTS** | |
|---|---|
| **Vial B - sterile solution** | |
| WATER FOR INJECTION | q.s. 1.5 ml |
| GLYCINE | 30.2 mg |
| L-PROLINE | 22.7 mg |
| L-LEUCINE | 4.2 mg |
| L-LYSINE HCL | 3.3 mg |
| L-VALINE | 16.8 mg |
| L-ALANINE | 22.8 mg |
| SODIUM SUCCINATE | 18 mg |
| SODIUM MALATE | 20.4 mg |

| **Ampoule (A) sterile solution** | |
|---|---|
| WATER FOR INJECTION | q.s. 1.5 ml |
| SODIUM HYALURONATE | 24 mg |

### Example 2

| **INGREDIENTS** | |
|---|---|
| **Vial B- freeze-dried powder** | |
| GLYCINE | 30.2 mg |
| L-PROLINE | 22.7 mg |
| L-LEUCINE | 4.2 mg |
| L-LYSINE HCL | 3.3 mg |
| L-VALINE | 16.8 mg |
| L-ALANINE | 22.8 mg |
| SODIUM SUCCINATE | 18 mg |
| SODIUM MALATE | 20.4 mg |

| **Ampoule (A) sterile solution** | |
|---|---|
| WATER FOR INJECTION | q.s. 3 ml |
| SODIUM HYALURONATE | 30 mg |

### Example 3

| **INGREDIENTS** | |
|---|---|
| **Ampoule - Sterile solution** | |
| WATER FOR INJECTION | q.s. 3.5 ml |
| SODIUM HYALURONATE | 35 mg |
| GLYCINE | 38.6 mg |
| L-PROLINE | 29.1 mg |
| L-LEUCINE | 5.4 mg |
| L-LYSINE HCL | 4.2 mg |
| L-VALINE | 21.5 mg |
| L-ALANINE | 29.2 mg |
| SODIUM SUCCINATE | 18 mg |
| SODIUM MALATE | 20.4 mg |

### Example 4 Pharmacological experiments

The most significant experiments conducted are described by way of example. In particular, the effects of a mixture of the 6 amino acids enriched with HA (6AAH), supplemented with succinate (6AAHS), malate (6AAHM) or succinate/malate (6AAHSM), on ECM gene expression in human fibroblasts were studied under both baseline conditions and in response to an oxidative stress (hydrogen peroxide).

BJ human fibroblasts were supplemented with 6AAH, 6AAHS, 6AAHM and 6AAHSM, and the mRNA levels of various ECM markers were evaluated. 6AAHSM increased the expression of all the ECM markers significantly more than 6AAH, either alone or plus succinate or malate only. Moreover, in an *in vitro* oxidative stress model, 6AAHSM attenuated the decline in ECM gene expression induced by hydrogen peroxide. The data suggest that diet supplementation with 6AAH enriched with malic and succinic acids is useful as a non-pharmacological approach to counteract skin aging.

### Materials and methods

### Cell cultures and treatment

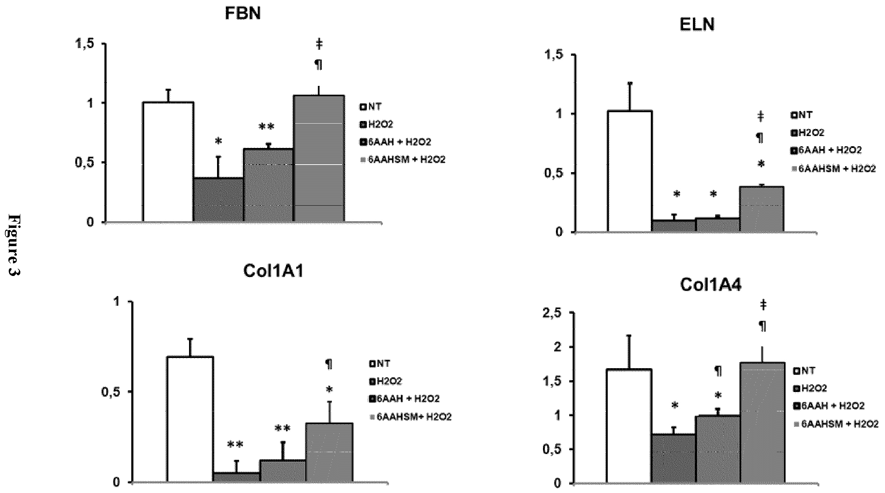

BJ human skin fibroblasts (ATCC^{®} CRL-2522^{™}) were cultured under standard conditions in EMEM medium (Sigma-Aldrich), supplemented with 10% foetal bovine serum (FBS) and antibiotics. The cell cultures were incubated at 37°C, with 95% humidity and 5% CO₂. The cells were treated with 0.1% (W/V) of the mixtures (the composition is shown in Table 1), +/- malic and succinic acid (5 mM each), for 3 days. The untreated cells were plated as control. Every 24 hours, the media were replaced with fresh media or mixtures of amino acids in both the control flasks and the treatment flasks. Oxidative stress with hydrogen peroxide was conducted on cells pre-treated with culture medium only or with the selected mixtures (Figure 3) for 24 hours; H₂O₂ (500 µM) was then added for 2 hours, and at the end of the experimental treatments, the cells were harvested for mRNA extraction.

**Table 1. Composition of the 6AAH mixture**

| **Ingredient** | **% of total** |
|---|---|
| Hyaluronic acid | 30 |
| Glycine | 30 |
| L-proline | 22.7 |
| L-leucine | 4.2 |
| L-lysine | 3.3 |
| L-valine | 16.8 |
| L-alanine | 22.8 |

### RNA extraction and quantitative RT-PCR

For the mRNA level analysis, 1 µg of total RNA, isolated with the RNeasy kit (Qiagen), was reverse-transcribed with the cDNA iScript synthesis kit (Bio-Rad Laboratories, Italy). PCR reactions in triplicate were conducted on an iCycler iQ Real-Time PCR detection system (Bio-Rad Laboratories). The corresponding gene expression was calculated with a comparative method (2-ΔΔCt) using GAPDH as housekeeping gene. The primer sequences were designed with Beacon Designer 2.6 software (Premier Biosoft International, Palo Alto, CA, USA).

### Protein extraction and western blotting:

The total proteins were extracted with M-PER Mammalian Protein Extraction Reagent (Pierce; ThermoScientific). The protein content was determined with the bicinchoninic acid (BCA) protein assay (Pierce); 30-40 µg of protein was analysed on sodium dodecyl sulphate-polyacrylamide TGX gel with a 4-20% gradient (SDS-PAGE) (BioRad). The gels were transferred to PVDF, blocked with 5% non-fat dried milk and incubated with anti-Fbn1 (1: 1000, GeneTex, Cat# GTX112794) or anti-vinculin (1:1000 Cat# V9131, Sigma-Aldrich).

### Description of Figures

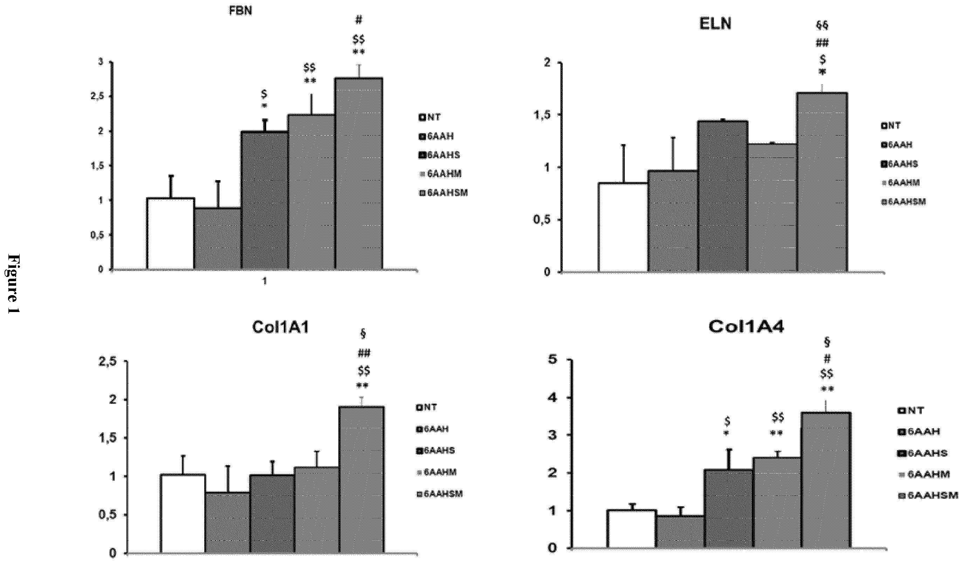

**Figure 1****:** Supplementation with malic/succinic acid increases mRNA expression of ECM markers. RT-PCR quantitative analysis of ECM gene expression in BJ fibroblasts. The data are mean ± SEM of samples in triplicate. NT: untreated controls, *P < 0.05 and **P < 0.01, vs NT, $ P < 0.05 and $ P < 0.01 vs 6AAH, #P < 0.05 and ##P < 0.01 vs 6AAHS, § P < 0.05 and §§ P < 0.01 vs 6AAHM. One-way ANOVA followed by Tukey's post hoc test.

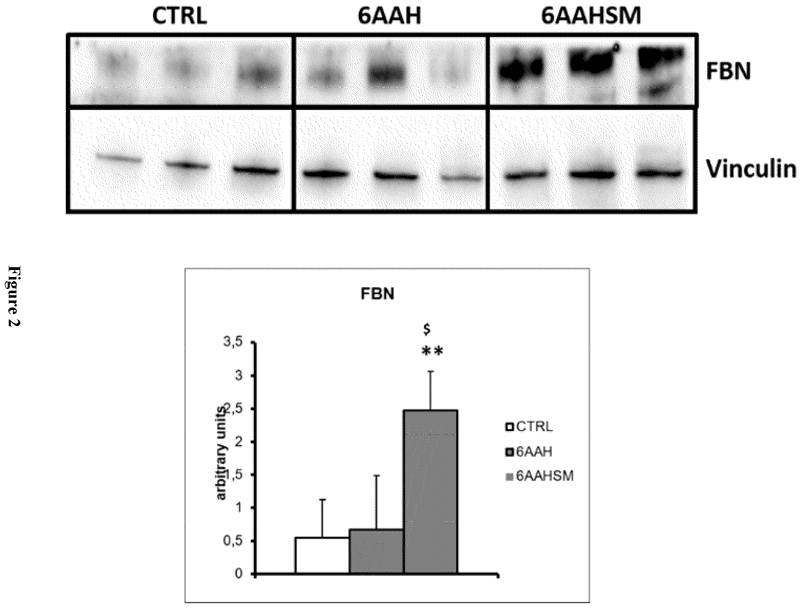

**Figure 2****:** Supplementation with 6AAHSM induces Fbn expression. Western blot analysis (top panel) of Fbn protein in BJ samples in triplicate. Vinculin was used as loading control. Bottom panel: quantitation of immunoblot data. CTRL: untreated controls, **P < 0.01, vs NT and $ P < 0.05 vs 6AAH. One-way ANOVA followed by Tukey's post hoc test.
**Figure 3****:** Supplementation with 6AAHSM protects ECM against oxidative stress. RT-PCR analysis of ECM markers in BJ fibroblasts treated with hydrogen peroxide. NT: untreated controls, hydrogen peroxide. The data are mean ± SEM of samples in triplicate. *P < 0.05 and **P < 0.01, vs NT, ¶ P < 0.05 vs H₂O₂, 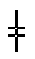 P < 0.05 vs 6AAH + H₂O₂. One-way ANOVA followed by Tukey's post hoc test.

### Results

The addition of malic and succinic acids to the mixture of 6 amino acids increases ECM gene expression to a significantly greater extent than amino acids alone.

The addition of succinic acid (6AAHS), malic acid (6AAHM) or succinic + malic (6AAHSM) acid to the 6AAH mixture increases expression of the ECM markers Fbn, Eln1, and of the two collagen isoforms col1a1 and col4a1 compared with 6AAH, 6AAHM or 6AAHS alone. Conversely, although supplementation with 6AAH alone (at a final concentration of 0.1% w/v) did not modify Fbn expression compared with the control cells (NT) (Fig. 1), the addition of succinate to the 6AAH mixture significantly increased expression of the mRNA levels of Fbn for both NTs and 6AAHs (+ 93% and +124% respectively). Supplementation of 6AAH with malate also increased Fbn expression in the 6AAHs (+ 152%) and in the controls (+116%). Similarly, supplementation of 6AAH with malate and succinate (6AAHSM) significantly increased Fbn expression compared with the controls (+167%) and with 6AAH (+211%); however, 6AAHSM also induced Fbn expression to a greater extent (+38%) than 6AAHS alone. Analysing Eln1 expression, only 6AAHSM increased its expression significantly compared with the controls (+101%) and the other mixtures, which were almost ineffective. Equally, expression of Col1A1 and Col4A1 was significantly increased by 6AAHSM; in particular, Col1A1 was significantly increased only by supplementation with 6AAHSM (+ 85% vs NT), whereas both 6AAHS and 6AAHM induced significant expression of Col1A4 (+106% and + 136% vs NT respectively); however, supplementation with 6AAHSM increased Col1A4 vs NT to a greater extent than 6AAHS and 6AAHM (+ 255%, + 72% and + 50% vs NT, 6AAHS and 6AAHM respectively).

It has been demonstrated that Fbn also plays a crucial part in regulating and promoting the deposition of various other components of ECM, including collagens (Sottile et al., 2007; Dalton and Lemmon, 2021). The protein expression of Fbn was therefore evaluated in BJ fibroblasts supplemented with culture media, 6AAH or 6AAHSM, which increased the mRNA of Fbn to a much greater extent than the other mixtures (Fig. 1). As shown in Fig. 2, 6AAHSM significantly increased expression of Fbn protein compared with BJ fibroblasts untreated or treated with 6AAH, thus further validating the mRNA expression data, and confirming that 6AAHSM regulates deposition of the components of ECM; moreover, supplementation of the 6AAH mixture with succinate and malate has a greater ability to induce ECM gene expression than supplementation of 6AAH with succinate or malate alone.

Having established that 6AAHSM was the most effective combination in inducing ECM gene expression, it was evaluated whether this ability also involves a greater ability to protect human fibroblasts against oxidative stress than that of the 6AAH mixture alone. For this purpose, BJ cells were pre-treated with H₂O₂ or H₂O₂ plus 6AAH, or H₂O₂ plus 6AAHSM, and ECM gene expression was evaluated. As expected, whereas treatment with H₂O₂ reduced the expression of all ECM genes, supplementation with 6AAH was ineffective in restoring their expression, with the exception of Co11A4, which was significantly increased by 6AAH compared with H₂O₂ only (Fig. 3). However, supplementation with 6AAHSM in H₂O₂ fibroblasts significantly reversed the oxidative stress-induced reduction of all ECM genes, with expression of FBN and Col1A4 completely restored to the control levels.

These data suggest that the addition of malic and succinic acids, either "as is" or in the form of pharmaceutically acceptable salts, increases the antioxidant capacity of the 6AAH mixture.

The results demonstrate that the composition according to the invention significantly stimulates ECM gene expression in human fibroblasts, even at concentrations below 0.1%.

Moreover, the composition according to the invention can reverse the downregulation of ECM gene expression induced by oxidative stress.

### Bibliography

Birch, H. L. (2018). in Subcellular Biochemistry, 169-190.
Black, L. D.,et al., .Biophys. J. 94, 1916-1929.
Chu, M. (2011). eLS*.*
Dalton, C. J., and Lemmon, C. A. (2021).Cells 10.
Langton, A. K. et al., (2010). Int. J. Cosmet. Sci. 32, 330-339.
Mohiuddin, A. K. (2019). Glob. J. Med. Res., 15-60.
Papakonstantinou, E. et al., (2012). Dermatoendocrinol. 4. doi: 10.4161/derm.21923.
Romani, et al., (2021). Nat. Rev. Mol. Cell Biol. 22, 22-38.
Rosso, F., (2004).. J. Cell. Physiol. 199, 174-180.
Scarano, A. et al. (2021). J. Cosmet. Dermatol. 20, 2296-2304. doi: 10.1111/jocd.13811.
Shin, J. W., et al. (2019). Int. J. Mol. Sci. 20.
Sottile, J. et al., (2007) Am. J. Physiol. Cell Physiol. 293. doi: 10.1152/ajpcell.00130.2007.
Sparavigna, A. (2020). Plast. Aesthetic Res. 2020. doi: 10.20517/2347-9264.2019.73.
Tedesco, L., et al. (2022). Biosci. Biotechnol. Biochem. 86, 1255-1261.
Theocharis, A. D., (2016). Adv. Drug Deliv. Rev. 97, 4-27.
Vuorio, E., and De Crombrugghe, B. (1990). Annu. Rev. Biochem. 59, 837-872.
Ziada, A. S., Smith, M. S. R., and Côté, H. C. F. (2020). Front. Cell Dev. Biol. 8*.*

## Claims

1. Compositions comprising:
a. an amino acid mixture consisting of glycine, L-proline, L-leucine, L-lysine, L-valine and L-alanine;
b. hyaluronic acid or salts thereof;
c. malic acid, succinic acid or the pharmaceutically acceptable salts thereof.

2. Compositions according to claim 1 wherein the weight ratios of the amino acids are:
- Glycine 1;
- L-Proline: 0.7-0.8;
- L-Alanine: 0.47-0.76;
- L-Valine: 0.35-0.56;
- L-Leucine: 0.13-0.27;
- L-Lysine hydrochloride: 0.10-0.12.

3. Compositions according to claim 1 or 2 wherein the weight ratio of malic acid to succinic acid is 1:1.

4. Compositions according to any one of claims 1 to 3 wherein the weight percentage of the mixture of amino acids is between 5 and 30%.

5. Compositions according to any one of claims 1 to 4 wherein the weight percentage of malic acid or a pharmaceutically acceptable salt thereof is between 1 and 5%.

6. Compositions according to any one of claims 1 to 5 wherein the weight percentage of succinic acid or a pharmaceutically acceptable salt thereof is between 1 and 5%.

7. Compositions according to any one of claims 1 to 6 wherein the hyaluronic acid or salts thereof, in particular sodium hyaluronate, has an average molecular weight ranging between 50,000 and 2,500,000 Da, in percentages ranging between 1 and 5% by weight of the total composition.

8. Compositions according to any one of claims 1 to 7 in the form of sterile solutions for injection or in a form suitable for topical administration.

9. The compositions of claims 1-8 for use in counteracting and preventing skin aging.
